# EUROPÄISCHE PATENTSCHRIFT

(11) **EP 2 281 044 B1**
(45) Veröffentlichungstag und Bekanntmachung des Hinweises auf die Patenterteilung: **09.05.2012**
(21) Anmeldenummer: 10721004.9
(22) Anmeldetag: 19.05.2010
(51) Int. Cl.: C12N 15/11

(54) **OLIGONUKLEOTIDE ZUR HEMMUNG UND ZUM NACHWEIS VON HUMANEN ARGONAUTE-PROTEINEN**
OLIGONUCLEOTIDES FOR INHIBITING AND FOR IDENTIFYING HUMAN ARGONAUTE PROTEINS
OLIGONUCLÉOTIDES DESTINÉS À INHIBER ET À DÉTECTER DES PROTÉINES ARGONAUTES HUMAINES

(30) Priorität: 12.06.2009 DE 102009024729; 12.06.2009 DE 102009024730; 12.06.2009 DE 102009024731; 12.06.2009 DE 102009024732
(43) Veröffentlichungstag der Anmeldung: 09.02.2011
(73) Patentinhaber: Universitätsklinikum Schleswig-Holstein Campus Lübeck, 23538 Lübeck (DE)
(72) Erfinder: SCZAKIEL, Georg, 23527 Gross-Grönau (DE); MESCALCHIN, Alessandra, 23564 Lübeck (DE); DETZER, Anke, 23562 Lübeck (DE); ENGEL, Christina, 53129 Bonn (DE); WEIRAUCH, Ulrike, 04229 Leipzig (DE); EROGULLARI, Alev, 23552 Lübeck (DE)
(74) Vertreter: Müller-Boré & Partner Patentanwälte
(86) Internationale Anmeldenummer: PCT/EP2010/003067
(87) Internationale Veröffentlichungsnummer: WO 2010/142371

(56) Entgegenhaltungen:
- WO-A2-2005/001031
- DATABASE EMBL [Online] 8. November 2002 (2002-11-08), "cs100c09.x1 Human Retinal pigment epithelium/choroid cDNA (Un-normalized, unamplified): cs Homo sapiens cDNA clone cs100c09 3', mRNA sequence." XP002592297 gefunden im EBI accession no. EMBL:CA389557 Database accession no. CA389557
- VICKERS T A ET AL: "Reduced levels of Ago2 expression result in increased siRNA competition in mammalian cells" NUCLEIC ACIDS RESEARCH, OXFORD UNIVERSITY PRESS, SURREY, GB LNKD- DOI:10.1093/NAR/GKM663, Bd. 35, Nr. 19, 28. September 2007 (2007-09-28), Seiten 6598-6610, XP002480333 ISSN: 0305-1048 [gefunden am 2007-09-28]
- MEISTER G ET AL: "Human Argonaute2 mediates RNA cleavage targeted by miRNAs and siRNAs" MOLECULAR CELL, CELL PRESS, CAMBRIDGE, MA, US LNKD- DOI:10.1016/J.MOLCEL.2004.07.007, Bd. 15, Nr. 2, 23. Juli 2004 (2004-07-23), Seiten 185-197, XP003016606 ISSN: 1097-2765

## Beschreibung

Die vorliegende Erfindung betrifft Oligonukleotide, die spezifisch an Argonaute-Proteine kodierende mRNAs hybridisieren können. Mit Hilfe dieser Oligonukleotide kann die Expression von Argonaute-Proteinen gehemmt und Argonaute-mRNA nachgewiesen werden. Die vorliegende Erfindung betrifft weiter diese Oligonukleotide enthaltende pharmazeutische Zusammensetzungen, Verfahren zur spezifischen Hemmung der Expression von Argonaute-Proteinen und zum Nachweis von Argonaute-mRNA, sowie die Verwendung dieser Oligonukleotide zur Hemmung der Expression von Argonaute-Proteinen in einer Zelle.

Die Familie der Argonaute (Ago)-Proteine ist eine hoch konservierte Proteinfamilie, deren Mitglieder Schlüsselfunktionen bei der RNA-vermittelten Interferenz (RNAi), d.h. dem durch kleine, nicht-kodierende RNAs vermittelten Gen-Silencing, innehaben. Ago-Proteine binden diese kleinen, nicht-kodierenden RNAs über ihre funktionellen Domänen, und können so unter anderem die Proteinsynthese kontrollieren oder die Stabilität von mRNA beeinflussen. Verschiedene Klassen von Ago-Proteinen lassen sich in allen höheren Eukaryonten finden, wo sie wichtige Funktionen in so verschiedenen Prozessen wie der Embryonalentwicklung, der Stammzellerneuerung oder der Zelldifferenzierung haben. Obwohl verschiedene Aspekte ihrer Biologie bereits erforscht wurden, sind viele Ago-Proteine noch immer nur sehr unzureichend charakterisiert.

Angesichts ihrer vielfältigen Funktionen werden Ago-Proteine auch mit verschiedenen Krankheiten in Verbindung gebracht. So konnte gezeigt werden, daß bestimmte Ago-Proteine mit FMRp interagieren, dem Gen, das bei dem vererbten Fragiles-X-Syndrom betroffen ist. Außerdem wurden einige Ago-Proteine mit der Regulation von Onkogenen und mit bestimmten Formen von Krebs assoziiert. Humanes Ago-1 ist ein Protein von 97,1 kDa. Es reguliert *small interfering RNA* (siRNA)-vermitteltes Silencing der Transkription durch Assoziation mit RNA Polymerase II (RNPII) und Induktion der Methylierung von Histonen, und es findet sich gehäuft in zytoplasmatischen Prozessierungskörperchen, den sogenannten P-Körperchen. Obwohl die Kristallstruktur der siRNA-bindenden Domäne von Ago-1, der sogenannten PAZ-Domäne, mit einem daran gebundenen doppelsträngigen siRNA-ähnlichen 9-mer bestimmt werden konnte, ist über die biologische Funktion von intaktem Ago-1 sehr wenig bekannt.

Humanes Ago-2 ist ein Protein von 97,25 kDa. Es ist eine Kernkomponente des RISC (*RNA-induced silencing comp*/*ex*), in dem es Funktionen hat, die mit der Hemmung und dem Abbau von mRNA in Zusammenhang stehen. Bindung von microRNA (miRNA) oder *small interfering* RNA (siRNA) an mRNA innerhalb des RISC führt entweder zu Hemmung der Translation oder Zerstörung der mRNA. Ago-2 ist bei den Säugetieren das einzige Ago-Protein, das eine mRNA spaltende Aktivität aufweist. Des Weiteren konnte gezeigt werden, daß Ago-2 ein zentraler Regulator der miRNA Homeostase ist, und regulatorische Funktionen bei der Frühentwicklung und der Hämatopoiese innehat. Dennoch ist über viele Aspekte der biologischen Funktion von Ago-2 noch wenig bekannt.

Humanes Ago-3 liegt in zwei Splicing-Varianten vor, der Isoform a und der Isoform b. Die Ago-3b kodierende mRNA und das entsprechende Protein sind kürzer als bei der Isoform a (Tabelle 1), wobei die verbleibende Sequenz bei beiden Varianten identisch ist. Über die biologische Funktion dieser Ago-3 Varianten ist sehr wenig bekannt.

**Tabelle 1: mRNA Länge und Molekulargewicht von Ago-3a und Ago-3b**

| | **mRNA Länge (b)** | **MW Protein (kDa)** |
|---|---|---|
| **Ago-3a** | 3578 | 97,4 |
| **Ago-3b** | 3320 | 71,2 |

Über humanes Ago-4 und seine biologischen Funktionen ist noch sehr wenig bekannt.

WO 2005/001031 A2 beschreibt antisense und RNAi Oligonukleotide mit denen die Expression von EIF2C1 (Argonaute-2) in Zellen verringert werden kann.

Der vorliegenden Erfindung liegt daher die Aufgabe zugrunde, neue und effektive Systeme für die Untersuchung von Argonaute-Proteinen und deren biologischen Funktionen, sowie für die Prävention und Behandlung von Störungen oder Krankheiten, die mit Argonaute-Proteinen in Zusammenhang stehen, bereitzustellen.

Diese Aufgabe wird durch die in den Ansprüchen gekennzeichneten Ausführungsformen der vorliegende Erfindung gelöst. Insbesondere werden erfindungsgemäß anti-sense Oligonukleotide (asONs) für die spezifische Hemmung der Expression von Argonaute-2-Proteinen bereitgestellt, die nicht auf RNA-vermittelter Interferenz (RNAi) basieren, sondern einen RNAse H-abhängigen Weg benutzen. Diese asONs zeichnen sich durch besonders effektives Assoziationsverhalten an Argonaute-mRNA aus.

Dementsprechend betrifft ein Gegenstand der vorliegenden Erfindung ein Oligonukleotid, umfassend eine oder mehrere der Sequenzen, ausgewählt aus der Gruppe, bestehend aus SEQ ID NO: 1 (5'-TATTCCTGCCCCCGTAGAG-3'), SEQ ID NO: 2 (5'-GTTTTGTGTTGCTTTCACTCTCAG-3'), SEQ ID NO: 3 (5'-GTGTTTTGT GTTGCTTTCACTCTC-3'), SEQ ID NO: 4 (5'-GTTTTGTGTTGCTTTCACTCTC-3'). Weitere Oligonukleotide wurden hergestellt. Diese sind SEQ ID NO: 14 (5'-GATCTTAGGGATGTCCACCTC-3'), SEQ ID NO: 15 (5'-CACCT CGTAGTGGTACACGT-3'), SEQ ID NO: 16 (5'-GCTTGGGCTGCTCATCTATG-3'), SEQ ID NO: 18 (5'-AGAAAAATGAACGCCCCACA-3'), SEQ ID NO: 19 (5'-CTTCTG GAGCGGAGAAAAAT-3'), SEQ ID NO: 20 (5'-CTAGTGGCAGGTAGGTGTGT-3'), SEQ ID NO: 21 (5'-GTGCAGGTATAGAAACAGAT-3'), SEQ ID NO: 22 (5'-CAGACT TCTAGTGGCAGG TA-3'), SEQ ID NO: 23 (5'-CCCTGCCACAATATTACAGACTT-3'), SEQ ID NO: 24 (5'-GTTGCCCTGCCACAATATTA-3'), SEQ ID NO: 30 (5'-CAG GCAAAGATTGGAAAGGG-3'), SEQ ID NO: 31 (5'-CAAGTTTTGCATTTATCTTC-3'), SEQ ID NO: 32 (5'-TGCAAGTAGTCGTGTGAGTT-3'), SEQ ID.NO: 33 (5'-AATT TGTACTGCAAGTAGTC-3'), SEQ ID NO: 34 (5'-GTTTATGAGATTTGGACCGT-3'), SEQ ID NO: 35 (5'-GAGAAGGCTAGTTTATGAGA-3').

Die erfindungsgemäßen Oligonukleotide betreffen das das Argonaute-Protein Argonaute-2 (Ago-2) und das erfindungsgemäße Oligonukleotid umfaßt eine oder mehrere der Sequenzen, ausgewählt aus der Gruppe bestehend aus SEQ ID NOs: 1, 2, 3, 4 und biologisch aktiven Derivaten davon.

Andere beschriebene Oligonukleotide betreffen das Argonaute-Protein Argonaute-1 (Ago-1) und diese Oligonukleotide umfassen eine oder mehrere der Sequenzen, ausgewählt aus der Gruppe bestehend aus SEQ ID NOs: 14, 15, 16 und biologisch aktiven Derivaten davon.

Andere beschriebene Oligonukleotide betreffen das Argonaute-Protein Argonaute-3 (Ago-3) und diese Oligonukleotide umfassen eine oder mehrere der Sequenzen, ausgewählt aus der Gruppe bestehend aus SEQ ID NOs: 18, 19, 20, 21, 22, 23, 24 und biologisch aktiven Derivaten davon. Desweiteren sind beschrieben die Sequenzen, ausgewählt aus SEQ ID NOs: 20, 21, 22, 23, 24 und biologisch aktiven Derivate davon. Hierbei liegt Ago-3 als Isoform Ago-3a oder Isoform Ago-3b vor. Liegt Ago-3 als Ago-3b vor, so umfaßt das Oligonukleotid eine oder mehrere der Sequenzen, ausgewählt aus SEQ ID NOs: 20, 21, 22, 23, 24 und biologisch aktiven Derivaten davon.

Andere beschriebene Oligonukleotide betreffen das Argonaute-Protein Argonaute-4 (Ago-4) und das erfindungsgemäße Oligonukleotid umfaßt eine oder mehrere der Sequenzen, ausgewählt aus der Gruppe bestehend aus SEQ ID NOs: 30, 31, 32, 33, 34, 35 und biologisch aktiven Derivaten davon.

Der Begriff "Oligonukleotid" bezeichnet ein natives, halbsynthetisches, synthetisches oder modifiziertes, einzel- oder doppelsträngiges Nukleinsäuremolekül aus Desoxyribonukleotiden und/oder Ribonukleotiden und/oder modifizierten Nukleotiden. Geeignete modifizierte Nukleotide umfassen Nukleotide mit modifizierten Basen, Zuckern und/oder Phosphatgruppen. Beispiele für modifizierte Nukleotide sind sogenannte LNAs (*locked nucleic acids*), bei denen das C2- und C4-Atom der Ribose über eine Sauerstoff-Methylen-Brücke verbunden ist, sowie Morpholino-Derivate von Nukleotiden, Nukleotid-Phosphorothioate, oder 2'-fluoro-und 2'-alkyl-modifizierte Nukleotide. Weiter können die erfindungsgemäßen Oligonukleotide beispielsweise als sogenannte "Gapmere" vorliegen, also als Oligonukleotide, bei denen beispielsweise ein zentraler Abschnitt aus Desoxyribonukleotiden gebildet ist, der von Abschnitten aus 2'-O-methylmodifizierten Ribonukleotiden flankiert ist.

Die Länge des erfindungsgemäßen Oligonukleotids unterliegt keinen besonderen Beschränkungen, wobei die Mindestlänge durch die Länge der Sequenzen SEQ ID NOs: 1 bis 4 vorgegeben ist. In einer bevorzugten Ausführungsform umfaßt das erfindungsgemäße Oligonukleotid 28 oder weniger, bevorzugt 23 oder weniger, besonders bevorzugt 18 oder weniger Nukleotide. In einer besonders bevorzugten Ausführungsform besteht das erfindungsgemäße Oligonukleotid aus einer der Sequenzen SEQ ID NOs: 1 bis 4. Verfahren zur Herstellung und/oder Isolierung von Oligonukleotiden sind dem Fachmann aus dem Stand der Technik bekannt und umfassen beispielsweise chemisch-synthetische oder enzymatische Verfahren, sowie die intrazelluläre Expression und nachfolgende Aufreinigung der Oligonukleotide.

Das erfindungsgemäße Oligonukleotid hat in einer bevorzugten Ausführungsform die Fähigkeit, spezifisch an Ago-2-mRNA zu binden bzw. zu hybridisieren. Die Hybridisierung wird bevorzugt bei Bedingungen, die den physiologischen Bedingungen im Zytosol oder Zellkern einer Zelle entsprechen, durchgeführt. In einer weiteren bevorzugten Ausführungsform wird die Hybridisierung bei Bedingungen, die für die *in vitro* Hybridisierung förderlich sind, durchgeführt. In einer besonders bevorzugten Ausführungsform erfolgt die Hybridisierung unter stringenten Bedingungen. Die genannten Bedingungen sind dem Fachmann aus dem Stand der Technik bekannt.

Das erfindungsgemäße Oligonukleotid kann Nukleotiddeletionen, -insertionen und/oder -substitutionen enthalten, solange die Fähigkeit, spezifisch an Ago-2-mRNA zu binden, im Wesentlichen erhalten bleibt. In einer weiteren bevorzugten Ausführungsform umfaßt das erfindungsgemäße Oligonukleotid mindestens eine modifizierte Nukleosidverknüpfung. In einer besonders bevorzugten Ausführungsform sind alle Nukleosidverknüpfungen modifiziert. Eine besonders bevorzugte modifizierte Nukleosidverknüpfung ist die Phosphorothioat-Verknüpfung.

Ein weiterer Gegenstand der vorliegenden Erfindung betrifft ein erfindungsgemäßes Oligonukleotid zur spezifischen Hemmung der Ago-2-Expression in einer Zelle. Der Begriff "Hemmung der Argonaute-Expression" bezeichnet eine Reduzierung der Expression von Argonaute-mRNA auf höchstens 50%, bevorzugt höchstens 35%, mehr bevorzugt höchstens 20% der normalen Expression von Argonaute-mRNA in einer Zelle.

In einer bevorzugten Ausführungsform wird die Hemmung der Ago-2. Expression bereits vier Stunden nach Transfektion einer Zelle mit dem entsprechenden erfindungsgemäßen Oligonukleotid erreicht. In einer weiteren bevorzugten Ausführungsform dauert die Hemmung der Ago-2. Expression bis zu 120 Stunden, mehr bevorzugt bis zu 72 Stunden und besonders bevorzugt bis zu 48 Stunden nach Transfektion einer Zelle mit dem entsprechenden erfindungsgemäßen Oligonukleotids an. In einer weiteren bevorzugten Ausführungsform wird die Hemmung der Ago-2. Expression in einer Zelle *in vitro* durchgeführt. Die Zelle, in der die Expression von Ago-2 gehemmt wird, ist bevorzugt eine humane Zelle.

Ein weiterer Gegenstand der vorliegenden Erfindung betrifft ein erfindungsgemäßes Oligonukleotid zur Herstellung einer diagnostischen oder pharmazeutischen Zusammensetzung. Die erfindungsgemäße pharmazeutische Zusammensetzung wird in einer bevorzugten Ausführungsform zur spezifischen Hemmung der Ago-2, Expression in einer Zelle verwendet. In einer weiteren bevorzugten Ausführungsform wird die erfindungsgemäße diagnostische oder pharmazeutische Zusammensetzung für die Prävention oder Behandlung einer Störung oder Krankheit, die mit Ago-2, in Zusammenhang steht, in einem Individuum verwendet. Ein weiterer Gegenstand der vorliegenden Erfindung betrifft die Verwendung des erfindungsgemäßen Oligonukleotids oder des erfindungsgemäßen Vektors zur Hemmung der Expression von Ago-2, in einer Zelle. Dabei wird in einer bevorzugten Ausführungsform die Expression von Ago-2 in einer Zelle *in vitro* gehemmt. Die oben genannten Ausführungsformen sind auch anwendbar auf die Oligonukleotide die Ago-1, Ago-3 und Ago-4 betreffen.

Die Störung oder Krankheit, die mit Ago-2 in Zusammenhang steht, ist in einer bevorzugten Ausführungsform eine Störung oder Krankheit der Angiogenese, der Hämatopoese, der Embryogenese oder der microRNA-Biogenese, Chorea Huntington oder die Entstehung von Tumoren, insbesondere Tumoren der weiblichen Brust.

Die Störung oder Krankheit, die mit Ago-1 in Zusammenhang steht, ist eine Störung oder Krankheit der Angiogenese, der Hämatopoese, der Embryogenese oder der microRNA-Biogenese, Chorea Huntington oder die Entstehung von Tumoren.

Die Störung oder Krankheit, die mit Ago-3 in Zusammenhang steht, ist eine Störung oder Krankheit der Angiogenese, der Hämatopoese, der Embryogenese oder der microRNA-Biogenese, Chorea Huntington oder die Entstehung von Tumoren.

Die Störung oder Krankheit, die mit Ago-4 in Zusammenhang steht, ist eine Störung oder Krankheit der Angiogenese, der Hämatopoese, der Embryogenese oder der microRNA-Biogenese, Chorea Huntington oder die Entstehung von Tumoren.

Das Individuum, das eine mit Ago-2 in Zusammenhang stehende Störung oder Krankheit aufweist, kann jedes Wirbeltier sein und ist in einer bevorzugten Ausführungsform ein Säuger, besonders bevorzugt ein Mensch.

Ein weiterer Gegenstand der vorliegenden Erfindung betrifft ein Nukleinsäure-Konstrukt, umfassend mindestens ein erfindungsgemäßes Oligonukleotid und mindestens eine detektierbare Markierung, sowie dessen Verwendung zum Nachweis von Ago-2, mRNA. In einer bevorzugten Ausführungsform wird die Ago-2, mRNA in einer Zelle nachgewiesen. In einer weiteren bevorzugten Ausführungsform wird die Ago-2, mRNA in einer Zelle *in vitro* nachgewiesen. Dies ist auch möglich für die Zusätzlich beschriebenen Ago-1, Ago-3 und Ago-4 oligonukleotide.

Der Begriff "detektierbare Markierung" beinhaltet geeignete, direkt oder indirekt nachweisbare Atome oder Moleküle, die in das erfindungsgemäße Oligonukleotid eingebaut und/oder daran gebunden sind. Geeignete Markierungen sind beispielsweise solche, die Fluoreszenzfarbstoffe und/oder beispielsweise Biotin und/oder Digoxigenin und/oder radioaktive Isotope umfassen. In einer bevorzugten Ausführungsform ist die detektierbare Markierung ein Fluoreszenzfarbstoff.

Ein weiterer Gegenstand der vorliegenden Erfindung betrifft einen Vektor, umfassend mindestens ein erfindungsgemäßes Oligonukleotid und/oder mindestens eine zum erfindungsgemäßen Oligonukleotid komplementäre Nukleinsäuresequenz, insbesondere DNA-Sequenz. Der erfindungsgemäße Vektor unterliegt keiner besonderen Einschränkung, wobei geeignete Vektoren im Stand der Technik bekannt sind. Der Vektor ist vorzugsweise zur Expression in einer eukaryontischen oder prokaryontischen Zelle befähigt. Dazu enthält der Vektor beispielsweise einen Promotor und gegebenenfalls geeignete regulatorische Elemente, wie Enhancer und Terminationssequenzen. Der Vektor kann auch zur stabilen Integration des erfindungsgemäßen Oligonukleotids in das genetische Material einer Zelle verwendet werden.

Ein weiterer Gegenstand der vorliegenden Erfindung betrifft eine Wirtszelle, welche mindestens ein erfindungsgemäßes Oligonukleotid oder mindestens einen erfindungsgemäßen Vektor enthält. Geeignete Wirtszellen sind beispielsweise Säugerzellen, insbesondere menschliche Zellen.

Des Weiteren wird erfindungsgemäß eine pharmazeutische Zusammensetzung, umfassend mindestens ein erfindungsgemäßes Oligonukleotid oder mindestens einen erfindungsgemäßen Vektor, bereitgestellt. Gegebenenfalls umfaßt die erfindungsgemäße pharmazeutische Zusammensetzung weiter einen oder mehrere pharmazeutisch verträgliche(n) Hilfsstoff(e) und/oder ein oder mehrere pharmazeutisch verträgliche(s) Verdünnungsmittel und/oder einen oder mehrere pharmazeutisch verträgliche(n) Träger. Die erfindungsgemäße pharmazeutische Zusammensetzung wird vorzugsweise zur präventiven oder therapeutischen Behandlung einer Störung oder Krankheit, die mit Ago-2 in Zusammenhang steht, verwendet. Besonders bevorzugte Einsatzgebiete sind die Behandlung von Störungen oder Krankheiten der Angiogenese, der Hämatopoese, der Embryogenese oder der microRNA-Biogenese, von Chorea Huntington oder von Tumoren, insbesondere Tumoren der weiblichen Brust. Dementsprechend betrifft ein Gegenstand der vorliegenden Erfindung eine pharmazeutische Zusammensetzung, umfassend mindestens ein erfindungsgemäßes Oligonukleotid oder mindestens einen erfindungsgemäßen Vektor, gegebenenfalls in Verbindung mit einem oder mehreren pharmazeutisch verträglichen Hilfsstoff(en) und/oder Verdünnungsmittel(n) und/oder Träger(n) zur Verwendung in der präventiven oder therapeutischen Behandlung einer Störung oder Krankheit, die mit Ago-2 in Zusammenhang steht, in einem Individuum. Die erfindungsgemäße pharmazeutische Zusammensetzung kann jegliche im vorliegenden Fachgebiet als geeignet betrachtete galenische Formen annehmen. Vorzugsweise ist sie fest, flüssig oder aerosolartig. Verabreichungsformen der erfindungsgemäßen pharmazeutischen Zusammensetzung unterliegen keinerlei bestimmten Beschränkungen. Geeignete Verabreichungsformen sind im Stand der Technik bekannt und umfassen beispielsweise orale, intravenöse, subkutane, intraperitoneale, intramuskuläre oder intratumorale Verabreichungsformen. Ebenso wird erfindungsgemäß eine diagnostische Zusammensetzung, umfassend mindestens ein erfindungsgemäßes Oligonukleotid oder mindestens ein erfindungsgemäßes Nukleinsäure-Konstrukt, bereitgestellt.

Ein weiterer Gegenstand der vorliegenden Erfindung betrifft ein Verfahren zur spezifischen Hemmung der Expression von Ago-2 in einer Zelle, umfassend das Einbringen mindestens eines erfindungsgemäßen Oligonukleotids oder mindestens eines erfindungsgemäßen Vektors in die Zelle und gegebenenfalls das Bereitstellen von geeigneten Hybridisierungsbedingungen, so daß die Expression von Ago-2 gehemmt wird. In einer bevorzugten Ausführungsform wird das erfindungsgemäße Verfahren zur spezifischen Hemmung der Expression von Ago-2 in einer Zelle *in vivo,* d.h. innerhalb des tierischen oder menschlichen Körpers durchgeführt. In einer weiteren bevorzugten Ausführungsform wird das genannte Verfahren *in vitro,* d.h. außerhalb des tierischen oder menschlichen Körpers durchgeführt. Verfahren zum Einbringen einer Nukleinsäure in eine Zelle sind im vorliegenden Fachgebiet wohlbekannt, und umfassen beispielsweise die Transfektion durch Elektroporation oder mit einem Transfektionsmittel wie z.B. Lipofectamin 2000. Die Hemmung der Ago-2. Expression in der Zelle hat vorzugsweise zur Folge, daß die RNA-vermittelte Interferenz (RNAi) der Zelle moduliert wird. In einer bevorzugten Ausführungsform ist die Zelle, deren Ago-2 Expression gehemmt werden soll, eine humane Zelle.

Ein weiterer Gegenstand der vorliegenden Erfindung betrifft ein Verfahren zum Nachweis von Ago-2 mRNA, umfassend das Inkontaktbringen des erfindungsgemäßen Konstrukts mit einer Ago-2 mRNA und das Bereitstellen von geeigneten Hybridisierungsbedingungen. Dieses Verfahren wird in einer bevorzugten Ausführungsform *in vitro* durchgeführt.

Ein weiterer Gegenstand der vorliegenden Erfindung betrifft ein Verfahren zum Nachweis von Ago-2 mRNA in einer Zelle, umfassend das Einbringen des erfindungsgemäßen Nukleinsäure-Konstrukts in eine Zelle, das Bereitstellen von geeigneten Hybridisierungsbedingungen und das Nachweisen der detektierbaren Markierung. Verfahren zum Nachweis der jeweiligen Markierung sind im vorliegenden Fachgebiet wohlbekannt. In einer bevorzugten Ausführungsform ist die Zelle, in der Ago-2 mRNA nachgewiesen werden soll, eine humane Zelle. Dieses Verfahren wird in einer bevorzugten Ausführungsform *in vitro* durchgeführt.

Ein weiterer Gegenstand der vorliegenden Erfindung betrifft einen Biochip/Microarray, umfassend ein oder mehrere immobilisierte(s) erfindungsgemäße(s) Oligonukleotid(e) und/oder ein oder mehrere immobilisierte(s) erfindungsgemäße(s) Nukleinsäure-Konstrukt(e). Der erfindungsgemäße Biochip/Microarray kann zum Nachweis von Ago-2 mRNA verwendet werden. Die vorliegende Erfindung umfaßt die Verwendung des erfindungsgemäßen Biochips/Microarrays zum Nachweis von Ago-2 mRNA.

Ein weiterer Gegenstand der vorliegenden Erfindung betrifft die Verwendung des erfindungsgemäßen Oligonukleotids oder des erfindungsgemäßen Nukleinsäure-Konstrukts für die Diagnose einer Störung oder Krankheit, die mit Ago-2 in Zusammenhang steht. Ein weiterer Gegenstand der vorliegenden Erfindung betrifft die Verwendung des erfindungsgemäßen Oligonukleotids oder des erfindungsgemäßen Vektors zur Behandlung einer Störung oder Krankheit, die mit Ago-2 in Zusammenhang steht.

Ein weiterer Gegenstand der vorliegenden Erfindung betrifft ein Verfahren zur Behandlung einer Störung oder Krankheit, die mit Ago-2 in Zusammenhang steht, in einem Individuum, umfassend das Verabreichen mindestens eines erfindungsgemäßen Oligonukleotids und/oder mindestens eines erfindungsgemäßen Vektors oder der erfindungsgemäßen pharmazeutischen Zusammensetzung an das Individuum.

Die Störung oder Krankheit, die mit Ago-2 in Zusammenhang steht, ist in einer bevorzugten Ausführungsform eine Störung oder Krankheit der Angiogenese, der Hämatopoese, der Embryogenese oder der microRNA-Biogenese, Chorea Huntington oder die Entstehung von Tumoren, insbesondere Tumoren der weiblichen Brust.

Die Störung oder Krankheit, die mit Ago-1 in Zusammenhang steht, ist eine Störung oder Krankheit der Angiogenese, der Hämatopoese, der Embryogenese oder der microRNA-Biogenese, Chorea Huntington oder die Entstehung von Tumoren.

Die Störung oder Krankheit, die mit Ago-3 in Zusammenhang steht, ist eine Störung oder Krankheit der Angiogenese, der Hämatopoese, der Embryogenese oder der microRNA-Biogenese, Chorea Huntington oder die Entstehung von Tumoren.

Die Störung oder Krankheit, die mit Ago-4 in Zusammenhang steht, ist eine Störung oder Krankheit der Angiogenese, der Hämatopoese, der Embryogenese oder der microRNA-Biogenese, Chorea Huntington oder die Entstehung von Tumoren.

Das Individuum, das eine mit Ago-2 in Zusammenhang stehende Störung oder Krankheit aufweist, ist in einer bevorzugten Ausführungsform ein Mensch. Ähnliche Überlegungen und Ausführungsformen sind denkbar für die zusätzlich beschriebenen Ago-1, Ago-3 und Ago-4 Oligonukleotide.

Die Figuren zeigen:
- Fig. 1:: Schematische Darstellung der Ago-2 mRNA mit den Bindungsstellen für SEQ ID NOs: 1 bis 13.
- Fig. 2:: Hemmung der Expression von humaner Ago-2 mRNA in ECV304 Zellen durch gegen Ago-2 mRNA gerichtete asONs. Die Expression von Ago-2 mRNA wurde gegen das Kontroll-Oligonukleotid asON scr (auch bezeichnet als T1-inv; SEQ ID NO: 36, 5'-TACCGCTCTTTTGACTTTTA-3') (schwarzer Balken), das auf 100% gesetzt wurde, normalisiert. Als Positivkontrolle (weißer Balken) kam das asON ISIS136764 (SEQ ID NO: 37, 5'-CTGCTGGAATGTTTCCACTT-3') zum Einsatz.
- Fig. 3:: Spezifität der Ago-2-spezifischen asONs gegenüber anderen humanen Argonaute-Proteinen (Agos). Die Expression der Ago mRNAs wurde gegen das Kontroll-Oligonukleotid asON scr, das auf 100% gesetzt wurde, normalisiert.
- Fig. 4:: Zeitkinetik der Hemmung der Expression von Ago-2 mRNA durch die asONs 1295/19 (SEQ ID NO: 1) und 3024/24 (SEQ ID NO: 3). Die Expression von Ago-2 mRNA wurde gegen das Kontroll-Oligonukleotid asON scr, das auf 100% gesetzt wurde, normalisiert.
- Fig. 5:: Schematische Darstellung der Ago-1 mRNA mit den Bindungsstellen für SEQ ID NOs: 14 bis 16.
- Fig. 6:: Hemmung der Expression von humaner Ago-1 mRNA in ECV304 Zellen durch gegen Ago-1 mRNA gerichtete asONs. Die Expression von Ago-1 mRNA wurde gegen das Kontroll-Oligonukleotid T1-inv, das auf 100% gesetzt wurde, normalisiert.
- Fig. 7:: Dosis-Wirkungs-Kurve für gegen Ago-1 mRNA gerichtete asONs. Die IC₅₀-Werte der asONs durch Transfektion von ECV304 Zellen mit aufsteigenden asON-Konzentrationen bestimmt. Die Daten der anschließenden quantitativen PCR wurden mit der Software GraFit 5 ausgewertet. Die Expression von Ago-1 mRNA wurde gegen das Kontroll-Oligonukleotid T1-inv, das auf 100% gesetzt wurde, normalisiert.
- Fig. 8:: Spezifität der Ago-1-spezifischen asONs gegenüber anderen humanen Argonaute-Proteinen (Agos). Die Expression der Ago mRNAs wurde gegen das Kontroll-Oligonukleotid T1-inv, das auf 100% gesetzt wurde, normalisiert.
- Fig. 9:: Zeitkinetik der Hemmung der Expression von Ago-1 mRNA durch das asON as343/21 (SEQ ID NO: 14). Die Expression von Ago-1 mRNA wurde gegen das Kontroll-Oligonukleotid T1-inv, das auf 100% gesetzt wurde, normalisiert.
- Fig. 10:: Schematische Darstellung der Ago-3a mRNA und der Ago-3b mRNA mit den Bindungsstellen für SEQ ID NOs: 18 bis 24. Die asONs asAgo3-1 und asAgo3-2 binden nur an Ago-3a mRNA, und die asONs asAgo3-6 und asAgo3-7 nur an Ago-3b mRNA. Alle anderen asONs binden an beide Isoformen.
- Fig. 11:: Hemmung der Expression von humaner Ago-3a mRNA (A) und Ago-3b mRNA (B) in ECV304 Zellen durch gegen Ago-3a mRNA und/oder Ago-3b mRNA gerichtete asONs. Die Expression von Ago-3a mRNA und Ago-3b mRNA wurde gegen das Kontroll-Oligonukleotid T1-inv, das auf 100% gesetzt wurde, normalisiert.
- Fig-12:: Spezifität der asONs asAgo3-1 und asAgo3-10 gegenüber anderen humanen Argonaute-Proteinen (Agos). Die Expression der Ago mRNAs wurde gegen das Kontroll-Oligonukleotid T1-inv, das auf 100% gesetzt wurde, normalisiert.
- Fig.13:: Zeitkinetik der Hemmung der Expression von Ago-3a mRNA und Ago-3b mRNA durch die asONs asAgo3-1 (SEQ ID NO: 18) und asAgo3-10 (SEQ ID NO: 24). Die Expression von Ago-3 mRNA wurde gegen das Kontroll-Oligonukleotid T1-inv, das auf 100% gesetzt wurde, normalisiert.
- Fig. 14:: Schematische Darstellung der Ago-4 mRNA mit den Bindungsstellen für SEQ ID NOs: 30 bis 35.
- Fig. 15:: Hemmung der Expression von humaner Ago-4 mRNA in ECV304 Zellen durch gegen Ago-4 mRNA gerichtete asONs. Die Expression von Ago-4 mRNA wurde gegen das Kontroll-Oligonukleotid T1-inv, das auf 100% gesetzt wurde, normalisiert.
- Fig. 16:: Spezifität der Ago-4-spezifischen asONs gegenüber anderen humanen Argonaute-Proteinen (Agos). Die Expression der Ago mRNAs wurde gegen das Kontroll-Oligonukleotid T1-inv, das auf 100% gesetzt wurde, normalisiert.
- Fig. 17:: Zeitkinetik der Hemmung der Expression von Ago-4 mRNA durch die asONs as1842/20 (SEQ ID NO: 30) und as4510/20 (SEQ ID NO: 34). Die Expression von Ago-4 mRNA wurde gegen das Kontroll-Oligonukleotid T1- inv, das auf 100% gesetzt wurde, normalisiert.

Die vorliegende Erfindung wird anhand der folgenden, nicht-einschränkenden Beispiele näher erläutert.

### Beispiel 1: Ago-2-spezifische anti-sense Oligonukleotide

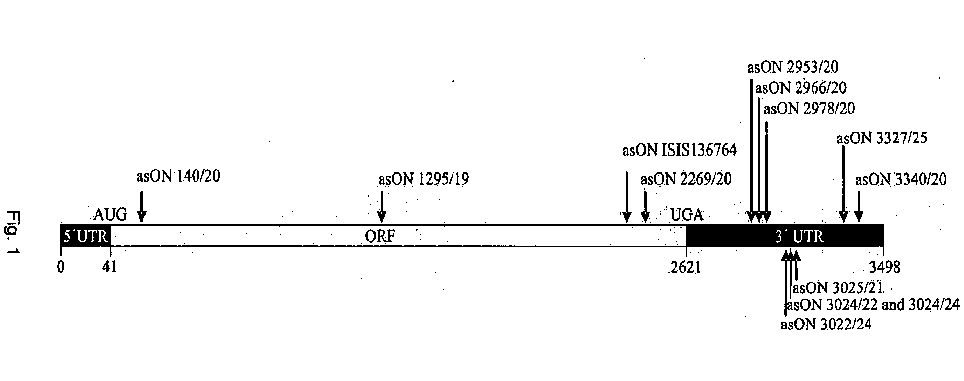

Es wurden 13 vollständig Phosphorothioat-modifizierte asONs getestet (Tabelle 2). Die Lage der Bindungsstellen der asONs an Ago-2 mRNA ist in Fig. 1 dargestellt. Die asONs sind gegen Bereiche des offenen Leserasters (*open reading frame*, ORF) der Ago-2 mRNA, und gegen Bereiche der 3' untranslatierten Region (3'-UTR) gerichtet.

**Tabelle 2: Getestete Ago-2-spezifische asONs.**

| **Name** | **Sequenz (5'nach 3')** | **SEQ ID NO** |
|---|---|---|
| asON 140/20 | AATTTGATTGTTCTCCCGGA | 5 |
| asON 1295/19 | TATTCCTGCCCCCGTAGAG | 1 |
| asON 2269/20 | GGTAGAAGTCGAACTCGGTG | 6 |
| asON 2953/20 | TTAAAAACAAGTAAATTGAA | 7 |
| asON 2966/20 | GAGAATCATGTATTAAAAAC | 8 |
| asON 2978/20 | ATCAATTTCATAGAGAATCA | 9 |
| asON ISIS136764 | CTGCTGGAATGTTTCCACTT | 10 |
| asON 3022/24 | GTTTTGTGTTGCTTTCACTCTCAG | 2 |
| asON 3024/24 | GTGTTTTGTGTTGCTTTCACTCTC | 3 |
| asON 3024/22 | GTTTTGTGTTGCTTTCACTCTC | 4 |
| asON 3025/21 | GTTTTGTGTTGCTTTCACTCT | 11 |
| asON 3340/20 | GATGATTAGACTGTCAGTAT | 12 |
| asON 3327/25 | GACTGTCAGTATATTGTCTTTTTTC | 13 |

### Beispiel 2: Hemmung humaner Ago-2 mRNA

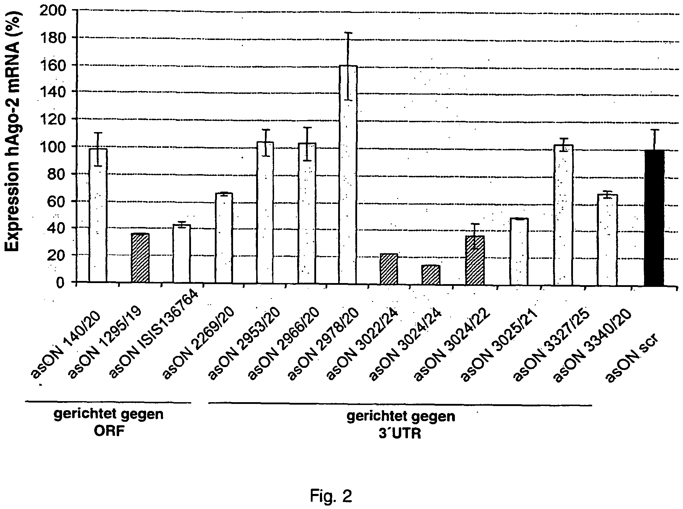

Die Fähigkeit der in Tabelle 1 gezeigten asONs, humane Ago-2 mRNA zu hemmen, wurde *in vitro* in der humanen Harnblasenkarzinomzelllinie ECV304 als Modellsystem getestet. ECV304 Zellen wurden mit 100 nM der spezifischen asONs durch Verwendung von Lipofectamin 2000 als Transfektionsmittel transfiziert. Nach 24 h in Kultur wurden die Zellen gesammelt, ihre RNA extrahiert, in cDNA umgeschrieben, und diese mittels quantitativer PCR amplifiziert. Die Wirkung der asONs auf die Ago-2 mRNA-Level ist in Fig. 2 gezeigt.

IC₅₀-Werte, d.h. die jeweilige asON-Konzentration, die benötigt wird, um 50% der Ago-2 mRNA Expression zu hemmen, wurden durch Transfektion von ECV304 Zellen mit aufsteigenden asON-Konzentrationen und nachfolgender quantitativer PCR bestimmt (Tabelle 3).

**Tabelle 3: IC₅₀-Werte**

| **Name** | **IC₅₀ (nM)** |
|---|---|
| asON 1295/19 | ≈ 70 |
| asON ISIS136764 | ≈ 90 |
| asON 3024/24 | ≈ 15 |

### Beispiel 3: Spezifität Ago-2-spezifischer asONs

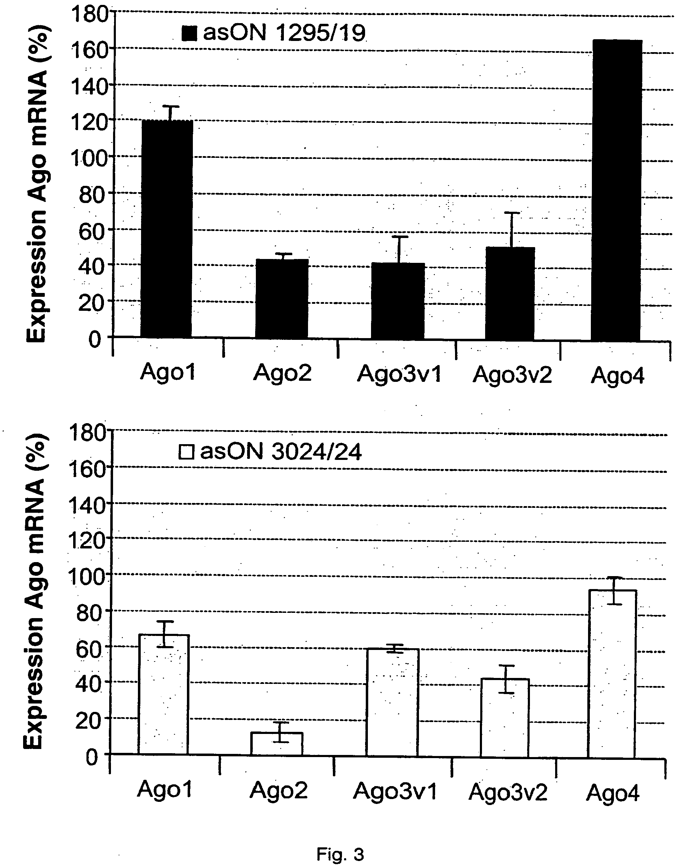

Die asONs 1295/19 und 3024/24 wurden auf ihre Spezifität für Ago-2 mRNA gegenüber anderen Ago mRNAs getestet. Die vier humanen Ago-Proteine weisen hoch homologe Sequenzen auf Nukleotid- und Peptidebene, mit bis zu 85% Aminosäureidentität, auf. 100 nM der asONs wurden in ECV304 Zellen mit Hilfe von Lipofectamin 2000 als Transfektionsmittel transfiziert. Nach 24 h in Kultur wurden die Zellen gesammelt, ihre RNA extrahiert, und in cDNA umgeschrieben. Die darauf folgende quantitative PCR wurde mit Primern für jedes Mitglied der humanen Ago-Familie durchgeführt. Die jeweiligen mRNA Expressionslevel für jedes Ago-Protein sind in Fig. 3 dargestellt.

### Beispiel 4: Kinetik der Ago-2 mRNA Hemmung

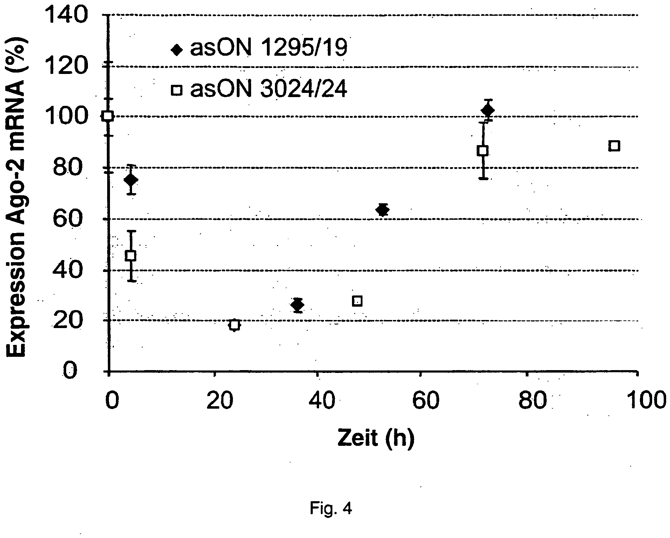

Die asONs 1295/19 und 3024/24 hemmen die Expression von Ago-2 mRNA bereits 4 h nach Transfektion. Dieser Effekt dauert bis zu 48 h nach Transfektion an (Fig. 4).

### Beispiel 5: Ago-1-spezifische anti-sense Oligonukleotide

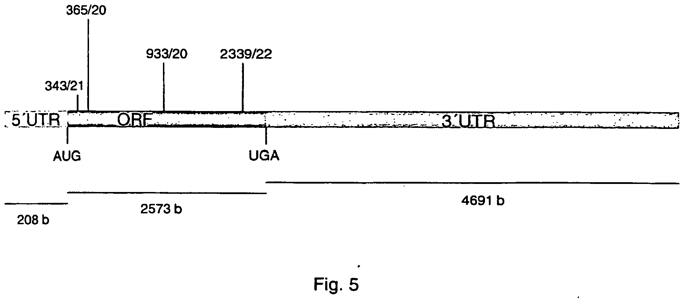

Es wurden vier vollständig Phosphorothioat-modifizierte asONs getestet (Tabelle 4). Die Lage der Bindungsstellen der asONs an Ago-1 mRNA ist in Fig. 5 dargestellt. Alle asONs sind gegen Bereiche des offenen Leserasters (*open reading frame*, ORF) der Ago-1 mRNA gerichtet.

**Tabelle 4: Getestete Ago-1-spezifische asONs.**

| **Name** | **Sequenz (5'nach 3')** | **SEQ ID NO** |
|---|---|---|
| as343/21 | GATCTTAGGGATGTCCACCTC | 14 |
| as365/20 | CACCTCGTAGTGGTACACGT | 15 |
| as933/20 | GCTTGGGCTGCTCATCTATG | 16 |
| as2339/22 | TAGCTCATAGTGGAGTATCTGG | 17 |

### Beispiel 6: Hemmung humaner Ago-1 mRNA

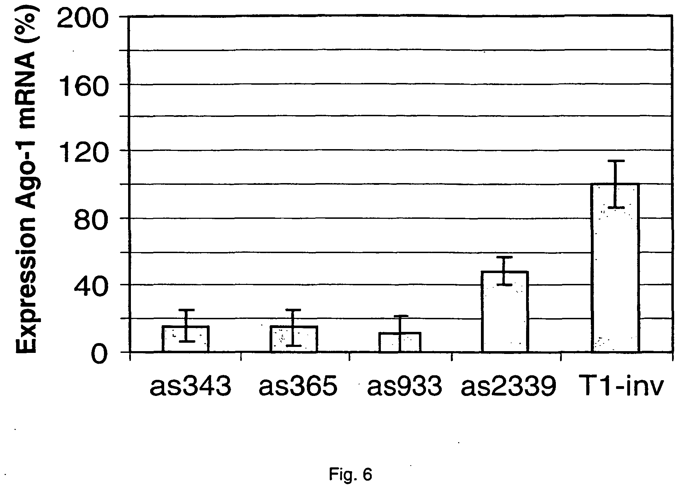

Die Fähigkeit der in Tabelle 3 gezeigten asONs, humane Ago-1 mRNA zu hemmen, wurde *in vitro* in ECV304 Zellen als Modellsystem getestet. ECV304 Zellen wurden mit 100 nM der spezifischen asONs durch Verwendung von Lipofectamin 2000 als Transfektionsmittel transfiziert. Nach 24 h in Kultur wurden die Zellen gesammelt, ihre RNA extrahiert, in cDNA umgeschrieben, und diese mittels quantitativer PCR amplifiziert. Die Wirkung der asONs auf die Ago-1 mRNA-Level ist in Fig. 6 gezeigt.

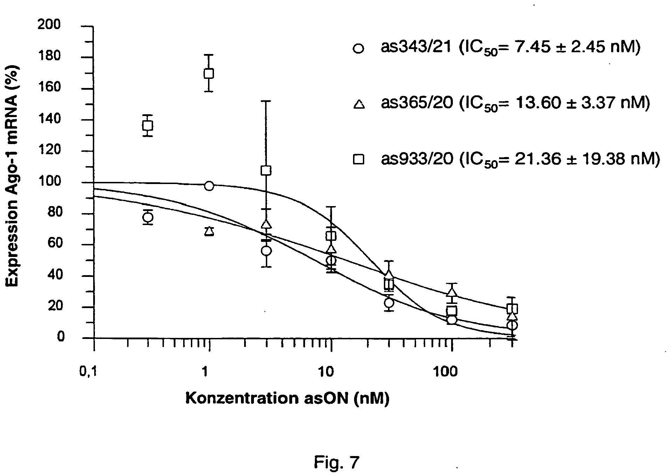

IC₅₀-Werte, d.h. die jeweilige asON-Konzentration, die benötigt wird, um 50% der Ago-1 mRNA Expression zu hemmen, wurden durch Transfektion von ECV304 Zellen mit aufsteigenden asON-Konzentrationen bestimmt. Nach quantitativer PCR wurden die Daten mit Hilfe der Software GraFit 5 ausgewertet (Fig. 7).

### Beispiel 7: Spezifität Apo-1-spezifischer asONs

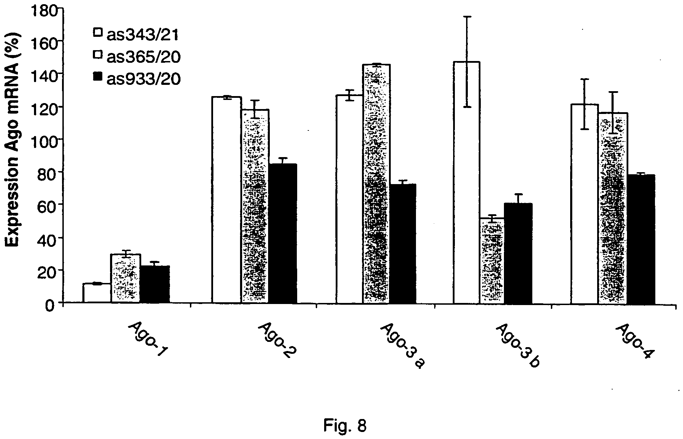

Alle asONs wurden auf ihre Spezifität für Ago-1 mRNA gegenüber anderen Ago mRNAs getestet. Die vier humanen Ago-Proteine weisen hoch homologe Sequenzen auf Nukleotid- und Peptidebene auf. 100 nM der asONs wurden in ECV304 Zellen mit Hilfe von Lipofectamin 2000 als Transfektionsmittel transfiziert. Nach der Transfektion wurde RNA extrahiert und in cDNA umgeschrieben. Die darauf folgende quantitative PCR wurde mit Primern für jedes Mitglied der humanen Ago-Familie durchgeführt. Die jeweiligen mRNA Expressionslevel für jedes Ago-Protein sind in Fig. 8 dargestellt.

### Beispiel 8: Kinetik der Ago-1 mRNA Hemmung

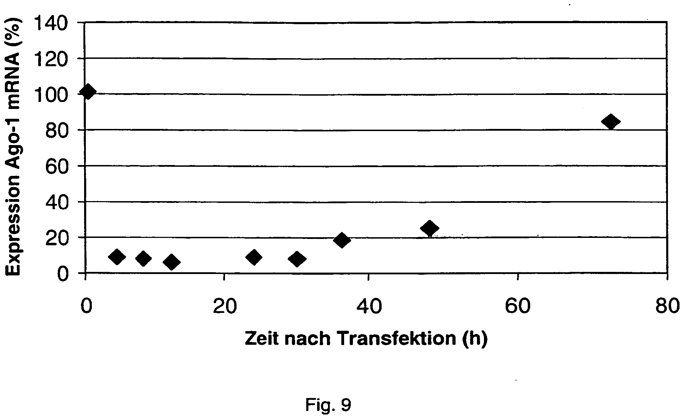

Das asON as343/21 hemmt die Expression von Ago-1 mRNA bereits 4 h nach Transfektion. Dieser Effekt dauert bis zu 72 h nach Transfektion an (Fig. 9).

### Beispiel 9: Apo-3-spezifische anti-sense Oligonukleotide

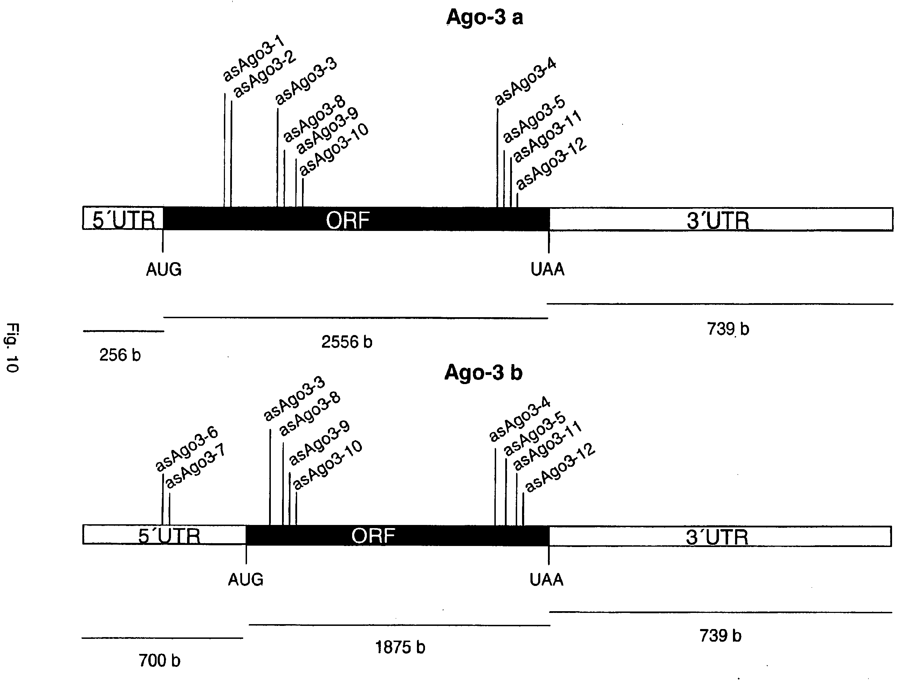

Es wurden zwölf vollständig Phosphorothioat-modifizierte asONs getestet (Tabelle 5). Die Lage der Bindungsstellen der asONs an Ago-3a mRNA bzw. Ago-3b mRNA ist in Fig. 10 dargestellt. Die asONs asAgo3-6 und asAgo3-7 sind gegen Bereiche der 5' untranslatierten Region (5'-UTR) der Ago-3b mRNA gerichtet. Die übrigen asONs sind gegen Bereiche des offenen Leserasters *(open reading frame,* ORF) der Ago-3a mRNA bzw. Ago-3b mRNA gerichtet.

**Tabelle 5: Getestete asONs.**

| **Ziel-mRNA** | **Name** | **Sequenz (5'nach 3')** | **SEQ ID NO** |
|---|---|---|---|
| Ago-3a | asAgo3-1 | AGAAAAATGAACGCCCCACA | 18 |
| | asAgo3-2 | CTTCTGGAGCGGAGAAAAAT | 19 |
| Ago-3b | asAgo3-6 | CGTCTAAATCTACCTGTTCACTCTC | 25 |
| | asAgo3-7 | AAGTAACGTCTAAATCTACCTGTTC | 26 |
| Ago-3a & Ago-3b | asAgo3-3 | CTAGTGGCAGGTAGGTGTGT | 20 |
| | asAgo3-4 | GCAGGTATAGAAACAGATCG | 27 |
| | asAgo3-5 | GTGCAGGTATAGAAACAGAT | 21 |
| | asAgo3-8 | CAGACTTCTAGTGGCAGGTA | 22 |
| | asAgo3-9 | CCCTGCCACAATATTACAGACTT | 23 |
| | asAgo3-10 | GTTGCCCTGCCACAATATTA | 24 |
| | asAgo3-11 | GTGCAGGTATAGAAACAGAT | 28 |
| | asAgo3-12 | CGCTGGTGCAGGTATAGAAA | 29 |

### Beispiel 10: Hemmung humaner Ago-3a mRNA bzw. Ago-3b mRNA

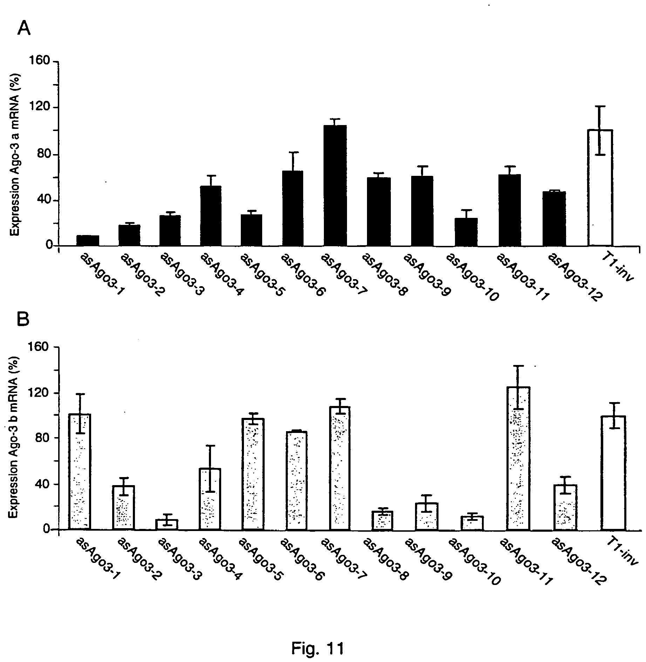

Die Fähigkeit der in Tabelle 5 gezeigten asONs, humane Ago-3a mRNA bzw. Ago-3b mRNA zu hemmen, wurde *in vitro* in ECV304 Zellen als Modellsystem getestet. ECV304 Zellen wurden mit 100 nM der spezifischen asONs durch Verwendung von Lipofectamin 2000 als Transfektionsmittel transfiziert. Nach 24 h in Kultur wurden die Zellen gesammelt, ihre RNA extrahiert, in cDNA umgeschrieben, und diese mittels quantitativer PCR amplifiziert. Die Wirkung der asONs auf die Ago-3a mRNA-Level bzw. Ago-3b mRNA-Level ist in Fig. 11 gezeigt.

IC₅₀-Werte, d.h. die jeweilige asON-Konzentration, die benötigt wird, um 50% der Ago-3a mRNA bzw. Ago-3b mRNA Expression zu hemmen, wurden durch Transfektion von ECV304 Zellen mit aufsteigenden asON-Konzentrationen bestimmt. Nach quantitativer PCR wurden die Daten mit Hilfe der Software GraFit 5 ausgewertet (Tabelle 6).

**Tabelle 6: IC₅₀-Werte**

| **Ziel mRNA** | **asON** | **IC₅₀ Ago-3a (nM)** | **IC₅₀ Ago-3b (nM)** |
|---|---|---|---|
| Ago-3a | asAgo3-1 | =10 | nd |
| Ago-3a & Ago-3b | asAgo3-3 | > 30 | ≈ 10 |
| | asAgo3-10 | =10 | ≈ 5 |

### Beispiel 11: Spezifität Ago-3-spezifischer asONs

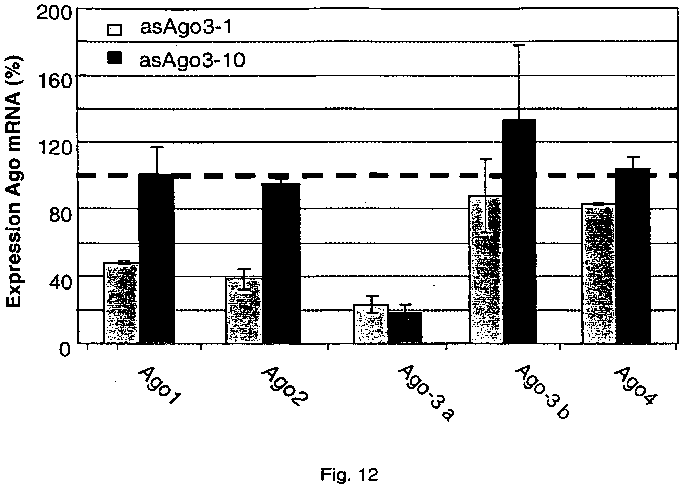

Die asONs asAgo3-1 und asAgo3-10 wurden auf ihre Spezifität für Ago-3a mRNA bzw. Ago-3b mRNA gegenüber anderen Ago mRNAs getestet. 100 nM der asONs wurden in ECV304 Zellen mit Hilfe von Lipofectamin 2000 als Transfektionsmittel transfiziert. Nach der Transfektion wurde RNA extrahiert und in cDNA umgeschrieben. Die darauf folgende quantitative PCR wurde mit Primern für jedes Mitglied der humanen Ago-Familie durchgeführt. Die jeweiligen mRNA Expressionslevel für jedes Ago-Protein sind in Fig. 12 dargestellt.

### Beispiel 12: Kinetik der Ago-3a mRNA und Ago-3b mRNA Hemmung

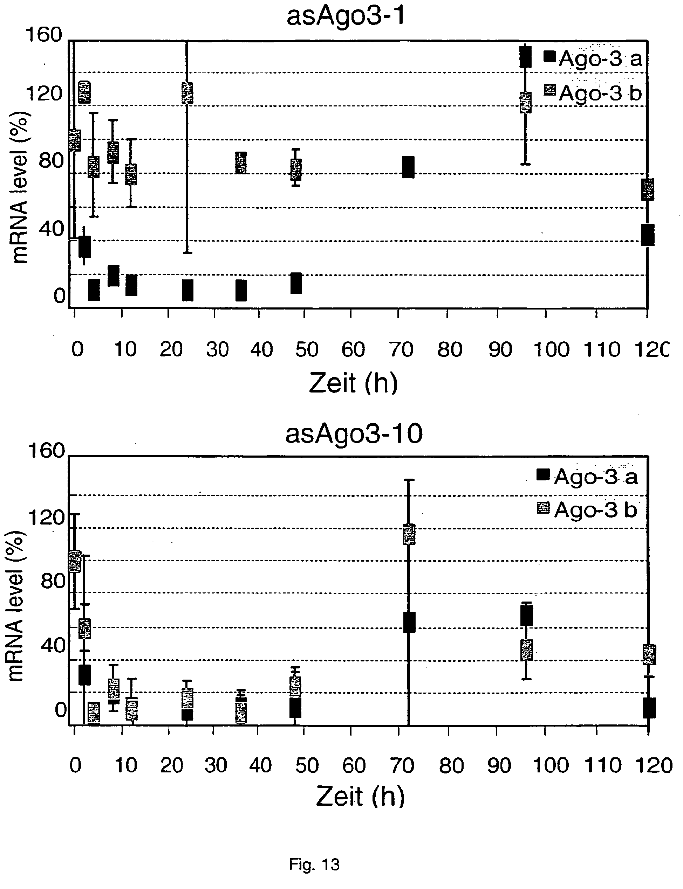

Das asON asAgo3-1 hemmt die Expression von Ago-3a mRNA bereits 4 h nach Transfektion. Dieser Effekt dauert bis zu 72 h nach Transfektion an. Das asON asAgo3-10 zeigt eine ähnliche Kinetik und wirkt auf beide Ago-3 mRNA Isoformen (Fig. 13).

### Beispiel 13: Ago-4-spezifische anti-sense Oligonukleotide

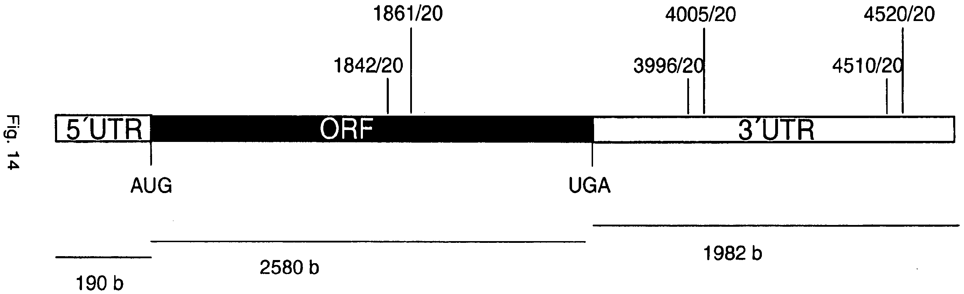

Es wurden sechs vollständig Phosphorothioat-modifizierte asONs getestet (Tabelle 7). Die Lage der Bindungsstellen der asONs an Ago-4 mRNA ist in Fig. 14 dargestellt. Die asONs as1842/20 und as1861/20 sind gegen Bereiche des offenen Leserasters (*open reading frame*, ORF) der Ago-4 mRNA gerichtet. Die übrigen asONs sind gegen Bereiche der 3' untranslatierten Region (3'-UTR) gerichtet.

**Tabelle 7: Getestete Ago-4-spezifische asONs.**

| **Name** | **Sequenz (5'nach 3')** | **SEQ ID NO** |
|---|---|---|
| as1842/20 | CAGGCAAAGATTGGAAAGGG | 30 |
| as1861/20 | CAAGTTTTGCATTTATCTTC | 31 |
| as3996/20 | TGCAAGTAGTCGTGTGAGTT | 32 |
| as4005/20 | AATTTGTACTGCAAGTAGTC | 33 |
| as4510/20 | GTTTATGAGATTTGGACCGT | 34 |
| as4520/20 | GAGAAGGCTAGTTTATGAGA | 35 |

### Beispiel 14: Hemmung humaner Ago-4 mRNA

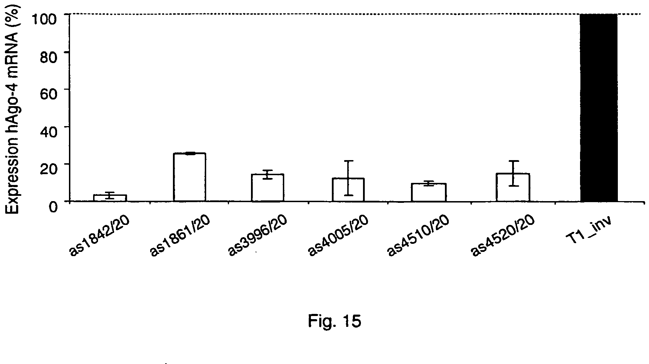

Die Fähigkeit der in Tabelle 7 gezeigten asONs, humane Ago-4 mRNA zu hemmen, wurde *in vitro* in ECV304 Zellen als Modellsystem getestet. ECV304 Zellen wurden mit 100 nM der spezifischen asONs durch Verwendung von Lipofectamin 2000 als Transfektionsmittel transfiziert. Nach 24 h in Kultur wurden die Zellen gesammelt, ihre RNA extrahiert, in cDNA umgeschrieben, und diese mittels quantitativer PCR amplifiziert. Die Wirkung der asONs auf die Ago-4 mRNA-Level ist in Fig. 15 gezeigt. IC₅₀-Werte, d.h. die jeweilige asON-Konzentration, die benötigt wird, um 50% der Ago-4 mRNA Expression zu hemmen, wurden durch Transfektion von ECV304 Zellen mit aufsteigenden asON-Konzentrationen bestimmt. Nach quantitativer PCR wurden die Daten mit Hilfe der Software GraFit 5 ausgewertet (Tabelle 8).

**Tabelle 8: IC₅₀-Werte**

| **asON** | **IC₅₀ (nM)** |
|---|---|
| as1842/20 | ≈ 15 |
| as4510/20 | ≈ 10 |

### Beispiel 15: Spezifität Ago-4-spezifischer asONs

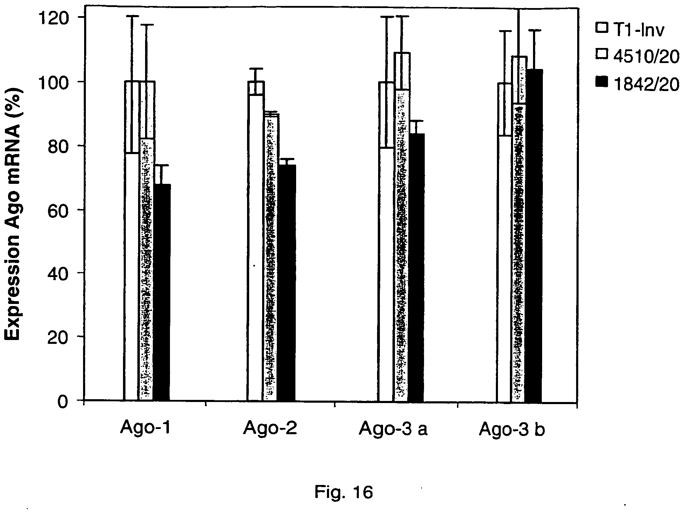

Die asONs as1842/20 und as4510/20 wurden auf ihre Spezifität für Ago-4 mRNA gegenüber anderen Ago mRNAs getestet. Die vier humanen Ago-Proteine weisen hoch homologe Sequenzen auf Nukleotid- und Peptidebene auf. 100 nM der asONs wurden in ECV304 Zellen mit Hilfe von Lipofectamin 2000 als Transfektionsmittel transfiziert. Nach der Transfektion wurde RNA extrahiert und in cDNA umgeschrieben. Die darauf folgende quantitative PCR wurde mit Primern für jedes Mitglied der humanen Ago-Familie durchgeführt. Die jeweiligen mRNA Expressionslevel für jedes Ago-Protein sind in Fig. 16 dargestellt.

### Beispiel 16: Kinetik der Ago-4 mRNA Hemmung

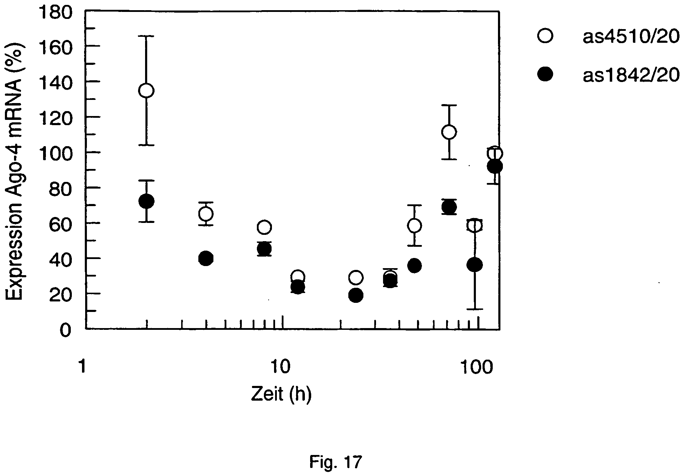

Die asONs as1842/20 und as4510/20 hemmen die Expression von Ago-4 mRNA bereits 4 h nach Transfektion. Dieser Effekt dauert bis zu 48 h nach Transfektion an (Fig. 17).

## Patentansprüche

1. Oligonukleotid, bestehend aus einer der Sequenzen, ausgewählt aus SEQ ID NOs: 1 bis 4, für die spezifische Hemmung der Expression von Argonaute-2 (Ago-2) in einer Zelle.

2. Oligonukleotid nach Anspruch 1, wobei die spezifische Hemmung der Expression von Argonaute-2 (Ago-2) in einer Zelle *in vitro* durchgeführt wird.

3. Oligonukleotid nach Anspruch 1 oder 2, wobei das Oligonukleotid mindestens eine modifizierte Nukleosidverknüpfung aufweist.

4. Oligonukleotid nach Anspruch 3, wobei die modifizierte Nukleosidverknüpfung eine Phosphorothioat-Verknüpfung ist.

5. Vektor, umfassend ein Oligonukleotid nach einem der Ansprüche 1 bis 4.

6. Nukleinsäure-Konstrukt, umfassend ein Oligonukleotid nach einem der Ansprüche 1 bis 4 und eine detektierbare Markierung.

7. Pharmazeutische Zusammensetzung, umfassend ein Oligonukleotid nach einem der Ansprüche 1 bis 4, gegebenenfalls in Verbindung mit einem oder mehreren pharmazeutisch verträglichen Hilfsstoff(en) und/oder Verdünnungsmittel(n) und/oder Träger(n).

8. Diagnostische Zusammensetzung, umfassend ein Oligonukleotid nach einem der Ansprüche 1 bis 4 oder das Nukleinsäure-Konstrukt nach Anspruch 6.

9. Verfahren zur spezifischen Hemmung der Expression von Argonaute-2 (Ago-2) in einer Zelle *in vitro,* umfassend das Einbringen eines Oligonukleotids nach einem der Ansprüche 1 bis 4 oder des Vektors nach Anspruch 5 in die Zelle und das Bereitstellen von geeigneten Hybridisierungsbedingungen.

10. Verfahren zum Nachweis von Argonaute-2 (Ago-2)-mRNA in einer Zelle *in vitro,* umfassend das Einbringen eines Nukleinsäure-Konstrukts nach Anspruch 6 in eine Zelle, das Bereitstellen von geeigneten Hybridisierungsbedingungen und das Nachweisen der detektierbaren Markierung.

11. Verwendung eines Oligonukleotids nach einem der Ansprüche 1 bis 4 zur Hemmung der Expression von Argonaute-2 (Ago-2) in einer Zelle *in vitro*.

12. Oligonukleotid nach einem der Ansprüche 1 bis 4 oder Nukleinsäure-Konstrukt nach Anspruch 6 zur Verwendung in der Diagnose einer Störung oder Krankheit, die mit Argonaute-2 (Ago-2) in Zusammenhang steht.

13. Oligonukleotid nach einem der Ansprüche 1 bis 4 zur Verwendung in der Behandlung einer Störung oder Krankheit, die mit Argonaute-2 (Ago-2) in Zusammenhang steht.

## Claims

1. Oligonucleotide composed of one of the sequences selected from SEQ ID Nos: 1 to 4 for the specific inhibition of the expression of argonaute-2 (Ago-2) in a cell.

2. Oligonucleotide according to claim 1, wherein the specific inhibition of the expression of argonaute-2 (Ago-2) in a cell is conducted *in vitro.*

3. Oligonucleotide according to claim 1 or 2, wherein the oligonucleotide has at least one modified nucleoside linkage.

4. Oligonucleotide according to claim 3, wherein the modified nucleoside linkage is a phosphorothioate linkage.

5. Vector comprising an oligonucleotide according to one of claims 1 to 4.

6. Nucleic acid construct comprising an oligonucleotide according to one of claims 1 to 4 and a detectable marking.

7. Pharmaceutical composition comprising an oligonucleotide according to one of claims 1 to 4, possibly in association with one or more pharmaceutically compatible auxiliary substances and/or diluting agents and/or carriers.

8. Diagnostic composition comprising an oligonucleotide according to one of claims 1 to 4 or the nucleic acid construct according to claim 6.

9. Method for the specific inhibition of the expression of argonaute-2 (Ago-2) in a cell *in vitro* comprising the incorporation of an oligonucleotide according to one of claims 1 to 4 or the vector according to claim 5 into the cell and the provision of suitable hybridisation conditions.

10. Method for the detection of argonaute-2 (Ago-2) mRNA in a cell *in vitro* comprising the incorporation of a nucleic acid construct according to claim 6 into a cell, the provision of suitable hybridisation conditions and the detection of the detectable marking.

11. Use of an oligonucleotide according to one of claims 1 to 4 for the specific inhibition of the expression of argonaute-2 (Ago-2) in a cell *in vitro.*

12. Oligonucleotide according to one of claims 1 to 4 or nucleic acid construct according to claim 6 for use in the diagnosis of a disorder or disease that is associated with argonaute-2 (Ago-2).

13. Oligonucleotide according to one of claims 1 to 4 for use in the treatment of a disorder or disease that is associated with argonaute-2 (Ago-2).

## Revendications

1. Oligonucléotide, consistant en une des séquences choisies parmi les SEQ ID N°1 à 4, pour l'inhibition spécifique de l'expression de argonaute-2 (Ago-2) dans une cellule.

2. Oligonucléotide selon la revendication 1, où l'inhibition spécifique de l'expression de argonaute-2 (Ago-2) dans une cellule est réalisée *in vitro.*

3. Oligonucléotide selon la revendication 1 ou 2, où l'oligonucléotide présente au moins une liaison de nucléoside modifiée.

4. Oligonucléotide selon la revendication 3, où la liaison de nucléoside modifiée est une liaison phosphorothioate.

5. Vecteur, comprenant un oligonucléotide selon l'une des revendications 1 à 4.

6. Construction d'acide nucléique, comprenant un oligonucléotide selon l'une des revendications 1 à 4 et un marqueur détectable.

7. Composition pharmaceutique, comprenant un oligonucléotide selon l'une des revendications 1 à 4, le cas échéant en combinaison avec un ou plusieurs auxiliaires et/ou agents de dilution et/ou supports pharmaceutiquement compatibles.

8. Composition diagnostique, comprenant un oligonucléotide selon l'une des revendications 1 à 4 ou la construction d'acide nucléique selon la revendication 6.

9. Procédé d'inhibition spécifique de l'expression de argonaute-2 (Ago-2) dans une cellule, *in vitro,* comprenant l'introduction d'un oligonucléotide selon l'une des revendications 1 à 4 ou du vecteur selon la revendication 5, dans la cellule et la préparation de conditions d'hybridation appropriées.

10. Procédé de détection de l'ARNm d'argonaute-2 (Ago-2) dans une cellule *in vitro,* comprenant l'introduction d'une construction d'acide nucléique selon la revendication 6 dans une cellule, la préparation de conditions d'hybridation appropriées et la détection du marqueur détectable.

11. Utilisation d'un oligonucléotide selon l'une des revendications 1 à 4 pour inhiber l'expression de l'argonaute-2 (Ago-2) dans une cellule *in vitro.*

12. Oligonucléotide selon l'une des revendications 1 à 4 ou construction d'acide nucléique selon la revendication 6 à utiliser dans le diagnostic d'un trouble ou d'une maladie, qui dépend de l'argonaute-2 (Ago-2).

13. Oligonucléotide selon l'une des revendications 1 à 4, à utiliser dans le traitement d'un trouble ou d'une maladie, qui dépend de l'argonaute-2 (Ago-2).


### SEQUENZPROTOKOLL

<110> universitätsklinikum schleswig-Holstein
<120> oligonukleotid zur Hemmung und zum Nachweis von humanen Argonaute-Proteinen
<130> L 1759WO
<160> 37
<170> PatentIn version 3.5
<210> 1
   <211> 19
   <212> DNA
   <213> künstliche Sequenz
<220>
   <223> anti-sense oligonukleotid
<400> 1
   tattcctgcc cccgtagag 19
<210> 2
   <211> 24
   <212> DNA
   <213> künstliche Sequenz
<220>
   <223> anti-sense oligonukleotid
<400> 2
   gttttgtgtt gctttcactc tcag 24
<210> 3
   <211> 24
   <212> DNA
   <213> künstliche Sequenz
<220>
   <223> anti-sense oligonukleotid
<400> 3
   gtgttttgtg ttgctttcac tctc 24
<210> 4
   <211> 22
   <212> DNA
   <213> künstliche Sequenz
<220>
   <223> anti-sense oligonukleotid
<400> 4
   gttttgtgtt gctttcactc tc 22
<210> 5
   <211> 20
   <212> DNA
   <213> künstliche Sequenz
<220>
   <223> anti-sense oligonukleotid
<400> 5
   aatttgattg ttctcccgga 20
<210> 6
   <211> 20
   <212> DNA
   <213> künstliche Sequenz
<220>
   <223> anti-sense oligonukleotid
<400> 6
   ggtagaagtc gaactcggtg 20
<210> 7
   <211> 20
   <212> DNA
   <213> künstliche Sequenz
<220>
   <223> anti-sense oligonukleotid
<400> 7
   ttaaaaacaa gtaaattgaa 20
<210> 8
   <211> 20
   <212> DNA
   <213> künstliche Sequenz
<220>
   <223> anti-sense oligonukleotid
<400> 8
   gagaatcatg tattaaaaac 20
<210> 9
   <211> 20
   <212> DNA
   <213> künstliche Sequenz
<220>
   <223> anti-sense oligonukleotid
<400> 9
   atcaatttca tagagaatca 20
<210> 10
   <211> 20
   <212> DNA
   <213> künstliche Sequenz
<220>
   <223> anti-sense oligonukleotid
<400> 10
   ctgctggaat gtttccactt 20
<210> 11
   <211> 21
   <212> DNA
   <213> künstliche Sequenz
<220>
   <223> anti-sense oligonukleotid
<400> 11
   gttttgtgtt gctttcactc t 21
<210> 12
   <211> 20
   <212> DNA
   <213> künstliche Sequenz
<220>
   <223> anti-sense oligonukleotid
<400> 12
   gatgattaga ctgtcagtat 20
<210> 13
   <211> 25
   <212> DNA
   <213> künstliche Sequenz
<220>
   <223> anti-sense Oligonukleotid
<400> 13
   gactgtcagt atattgtctt ttttc 25
<210> 14
   <211> 21
   <212> DNA
   <213> künstliche Sequenz
<220>
   <223> anti-sense oligonukleotid
<400> 14
   gatcttaggg atgtccacct c 21
<210> 15
   <211> 20
   <212> DNA
   <213> künstliche Sequenz
<220>
   <223> anti-sense Oligonukleotid
<400> 15
   cacctcgtag tggtacacgt 20
<210> 16
   <211> 20
   <212> DNA
   <213> künstliche Sequenz
<220>
   <223> anti-sense oligonukleotid
<400> 16
   gcttgggctg ctcatctatg 20
<210> 17
   <211> 22
   <212> DNA
   <213> künstliche Sequenz
<220>
   <223> anti-sense oligonukleotid
<400> 17
   tagctcatag tggagtatct gg 22
<210> 18
   <211> 20
   <212> DNA
   <213> künstliche Sequenz
<220>
   <223> anti-sense oligonukleotid
<400> 18
   agaaaaatga acgccccaca 20
<210> 19
   <211> 20
   <212> DNA
   <213> künstliche Sequenz
<220>
   <223> anti-sense oligonukleotid
<400> 19
   cttctggagc ggagaaaaat 20
<210> 20
   <211> 20
   <212> DNA
   <213> künstliche Sequenz
<220>
   <223> anti-sense oligonukleotid
<400> 20
   ctagtggcag gtaggtgtgt 20
<210> 21
   <211> 20
   <212> DNA
   <213> künstliche Sequenz
<220>
   <223> anti-sense oligonukleotid
<400> 21
   gtgcaggtat agaaacagat 20
<210> 22
   <211> 20
   <212> DNA
   <213> künstliche Sequenz
<220>
   <223> anti-sense oligonukleotid
<400> 22
   cagacttcta gtggcaggta 20
<210> 23
   <211> 23
   <212> DNA
   <213> künstliche Sequenz
<220>
   <223> anti-sense oligonukleotid
<400> 23
   ccctgccaca atattacaga ctt 23
<210> 24
   <211> 20
   <212> DNA
   <213> künstliche Sequenz
<220>
   <223> anti-sense oligonukleotid
<400> 24
   gttgccctgc cacaatatta 20
<210> 25
   <211> 25
   <212> DNA
   <213> künstliche Sequenz
<220>
   <223> anti-sense oligonukleotid
<400> 25
   cgtctaaatc tacctgttca ctctc 25
<210> 26
   <211> 25
   <212> DNA
   <213> künstliche Sequenz
<220>
   <223> anti-sense oligonukleotid
<400> 26
   aagtaacgtc taaatctacc tgttc 25
<210> 27
   <211> 20
   <212> DNA
   <213> künstliche Sequenz
<220>
   <223> anti-sense oligonukleotid
<400> 27
   gcaggtatag aaacagatcg 20
<210> 28
   <211> 20
   <212> DNA
   <213> künstliche Sequenz
<220>
   <223> anti-sense oligonukleotid
<400> 28
   gtgcaggtat agaaacagat 20
<210> 29
   <211> 20
   <212> DNA
   <213> künstliche Sequenz
<220>
   <223> anti-sense oligonukleotid
<400> 29
   cgctggtgca ggtatagaaa 20
<210> 30
   <211> 20
   <212> DNA
   <213> künstliche Sequenz
<220>
   <223> anti-sense oligonukleotid
<400> 30
   caggcaaaga ttggaaaggg 20
<210> 31
   <211> 20
   <212> DNA
   <213> künstliche Sequenz
<220>
   <223> anti-sense oligonukleotid
<400> 31
   caagttttgc atttatcttc 20
<210> 32
   <211> 20
   <212> DNA
   <213> künstliche Sequenz
<220>
   <223> anti-sense Oligonukleotid
<400> 32
   tgcaagtagt cgtgtgagtt 20
<210> 33
   <211> 20
   <212> DNA
   <213> künstliche Sequenz
<220>
   <223> anti-sense oligonukleotid
<400> 33
   aatttgtact gcaagtagtc 20
<210> 34
   <211> 20
   <212> DNA
   <213> künstliche Sequenz
<220>
   <223> anti-sense oligonukleotid
<400> 34
   gtttatgaga tttggaccgt 20
<210> 35
   <211> 20
   <212> DNA
   <213> künstliche Sequenz
<220>
   <223> anti-sense oligonukleotid
<400> 35
   gagaaggcta gtttatgaga 20
<210> 36
   <211> 20
   <212> DNA
   <213> künstliche Sequenz
<220>
   <223> Kontroll-Oligonukleotid
<400> 36
   taccgctctt ttgactttta 20
<210> 37
   <211> 20 <212> DNA
   <213> künstliche Sequenz
<220>
   <223> Positivkontroll-Oligonukleotid
<400> 37
   ctgctggaat gtttccactt 20